# EUROPEAN PATENT APPLICATION

(11) **EP 0 997 732 A1**
(43) Date of publication of application: **03.05.2000**
(21) Application number: 99203475.1
(22) Date of filing: 22.10.1999
(51) Int. Cl.: G01N 33/18

(54) **Method and equipment for continuous measurement of mineral oils in water by means of spectrophotometric detector**

(30) Priority: 29.10.1998 IT MI982315
(71) Applicant: ENEL S.p.A., I-00198 Roma (IT)
(72) Inventor: Achilli, Marco, 29100 Piacenza (IT); Balconi, Maria Luisa, 20132 Milano (IT); Gacs, Istvan, 1026 Budapest (HU); Martinotti, Valter, 22050 Verderio S., (LC) (IT); Sigon, Fabio, 20065 Inzago, (MI) (IT)
(74) Representative: Mittler, Enrico

(57) **Abstract**

There are described a method and an equipment for continuous measurement of mineral oils in water. The method provides for a feeding step in which a fluid to be analysed is pumped into a sampling device (2) in which the oils that are present in the fluid are separated by retention of the same inside said sampling device (2), and an elution and measurement step, in which the oils that are retained in said sampling device (2) undergo elution and they are transferred to a spectrophotometric detector (8) by means of circulation of the solvent in a closed circuit (6), comprising said sampling device (2), said spectrophotometric detector (8) and a device for the regeneration (9) of the solvent, in a sequence. The equipment for the realisation of the aforementioned method provides for commutation means (3) to alternately insert said sampling device (2) in a circuit for the feeding and draining of the fluid to be analysed and in a solvent circulation closed circuit (6) including the detector (8) and the regeneration circuit (9).

## Description

The present invention concerns a method and an equipment for continuous measurement of mineral oils in water by means of spectrophotometric detector.

It can be applied to the determination of oils in waters in general, for instance in the quality control of waste waters in industrial plants.

The analysis of oils in water has a particular environmental interest, also with reference to the regulations in matter of wastes, for the protection of waters from pollution.

Industrial processes can be responsible for the dumping of oils and, therefore, they must be controlled, possibly in a continuous and automatic way.

The polluting effect is evident not only in the alteration of the chemical quality of water, but also through the alteration of the processes of surface oxygen exchange, thus causing a decreased oxygenation with consequent damages in the aquatic life.

Besides, the control of oils is required in the case of a recovery, also partial, of the waters contaminated with oils within the production process, possibly in the sphere of a system for the treatment of the same waters.

The principal characteristics that a method of oils measurement must have are the following:
a) high frequency of measurement or possibility to take measurements in continuous, with the aim of being able to quickly predispose possible emergency interventions (for instance to resolve pollution problems);
b) easiness and simplicity of operation, in order for the method to be utilisable on plant, even in critical conditions;
c) low environmental impact and, therefore, a reduced or null use of reactants (ex. organic solvents) capable to produce wastes of substances that are potentially harmful to the environment;
d) sufficient sensitivity, for the control of the compliance with the limits established by current standards or by specifications for the reuse in the productive process, and absence or reduction of the interferences;
e) possibility to work automatically, with reduced external interventions on behalf of the operator;
f) possibility to utilise analytical methodologies that use the same method of measurement provided by the current technical standards.

The main methods and the main analytical techniques currently available for the analysis of oils in water are:
a) gravimetry, that provides for the determination of oils after extraction with solvent, and evaporation of the latter by weigh;
b) infrared spectrophotometry (IR), that provides for the measurement by absorption in the infrared of the bands related to the carbon-hydrogen aliphatic and aromatic bonds, after extraction with solvent;
c) spectrofluorimetry, that allows to determine the concentration of oils through the measurement of the released fluorescence, both on the sample as it is as well as after extraction with solvent;
d) UV absorption, that provides for the measurement on the sample as it is but that suffers considerable problems of interferences;
e) diffusion, after homogenisation of the sample with the purpose of obtaining very dispersed emulsions;
f) surface reflection, that provides for the separation in the form of a layer of the oil present in the sample taken.

For each one of these techniques there are different types of instruments, essentially in the automatic version.

The automation of the techniques is in fact possible, but it generally involves a certain degree of complication with consequent considerable employment of resources in managing and maintenance activity in addition to a reduced reliability of the measurement.

In addition the techniques that provide for a step of extraction with solvent involve problems with the disposal the used solvent as well as the handling of the same solvent, aspect that is particularly important in the case of the chlorinated solvents, because of their toxicity. Often the quantities of used solvent to be disposed of are high, with a considerable negative impact on final costs.

Finally, analysis times are often incompatible with the need for a high frequency monitoring and the limits of sensitivity sometimes are not congruent with the limits set by the law for the protection of waters from pollution, or with the limits required for the reutilisation of treated waters.

Object of the present invention is to realise a method and an equipment for the spectrophotometric analysis (that is with the use of spectrofluorimetric techniques or by means of IR spectrophotometry) of oils in water, that is free of the aforesaid drawbacks, that is a method and an equipment that allow to determine the concentration of oils in water by means of automatic measurements, having considerable sensitivity, high frequency of repetition, extremely reduced environmental impact, and characterized by extreme operating simplicity and low costs.

According with the invention such object has been attained with a method for continuous measurement of mineral oils in water, characterized in that it comprises, alternate to each other, a feeding step, in which a fluid to be analysed is pumped into a sampling device in which the oils present in the fluid are separated by retention of the same on the inside surfaces of said sampling device, and an elution and measurement step, in which the oils that are retained in said sampling device undergo elution and are transferred to a spectrophotometric detector by means of circulation of the solvent in a closed circuit, comprising said sampling device, said spectrophotometric detector and a device for the regeneration of the solvent, in a sequence.

In a preferred embodiment the method according to the present invention provides the following steps:
- feeding of the fluid in the sampling device (for example consisting of a steel capillary, with variable volume 0,1-5 ml, with or without packing material), that can be carried out by means of a pump capable to guarantee a flow of about 0,5-5 ml/ min. (for example: peristaltic pump);
- retention of the oils inside, that is on the inside surfaces, of said sampling device, connected with means for the control of the flow, preferably consisting of a multiple way injection valve (for instance six-way);
- elution of the oils retained in the sampling device by means of adequate organic solvent (for instance n-hexane), that is pumped into a closed circuit by means of a pump capable to guarantee a flow of about 1-3 ml/ min. (example: pump for liquid chromatography);
- measurement of the concentration of oils in the organic phase for by spectrofluorimetry, by means of flow cell detector with adjustable excitation and emission wavelength (for example, 300 nm and 350 nm respectively). The spectrofluorimetric detector can be replaced by an IR detector: in this case, using Freon as a solvent, a system capable to operate in compliance with the current technical standard (EPA 418-1; ASTM D 3921-85) is achieved;
- recording of the instrument signal through recorder, integrator or personal computer.

The method according to the invention allows the determination of low concentrations of oils in water (mg/ L). It is based on the retention of oils inside a sampling device that can be made up of a pipe, for instance an adequately shaped capillary of steel (pipe coil), with or without packing material (consisting for example of microspheres of steel or of resins), on the subsequent elution of the oils with organic solvent and on the in-flow measurement by means of spectrophotometric technique.

The proposed method therefore follows the general operation principle of in flow analysis methodologies, in which the sample is transported in a carrier flow (organic solvent, in this case) toward the detector, where the measurement takes place.

As compared with the known methods, it resolves the problem of the continuous monitoring at high frequency, thus reducing the times required for the completion of a single analysis, while maintaining a considerable operating and instrumental simplicity and a good analytical sensitivity. In addition, since the quantities of solvent necessary for the extraction of oils are minimal and also the recovery and the regeneration of the same in closed cycle are carried out, the problem connected with the disposal or with the recovery of the high quantity of organic solvents that is encountered with the other currently available methods is resolved positively.

In substance the method according to the present invention shows the undoubted advantages, that can be summed up as follows:
a) considerable sensitivity, deriving from the typical high sensitivity of the spectrophotometric measurement technique being used. It allows to detect concentrations in the order of the mg/ L of oils; in addition, it is possible to vary the dimension or the packing of the sampling device in order to additionally lower the limit of detectability;
b) extremely reduced consumption of reactants owing to the technique of recycling of the solvent. The solvent can be reused by recycling, in a closed circuit comprising a tank of about 1 L, for hundreds of analysis. The deviation of the base line because of the increase in concentration of oils in the solvent is negligible for such number of analysis, and it is controlled by means of treatment of purification of the same solvent in the return lines to the tank;
c) reduced problems in the handling of toxic solvents. The technique utilised allows, for a high number of analysis, the use of low quantities of solvent (about 1 L), that in addition is made circulate in a closed circuit and therefore reused;
d) rapidity of analysis, obtained owing to the alternation, automatically realisable, of the steps of feeding with the ones of elution and measurement, without dead times deriving from the need to feed and unload the sample from the sampling device. With this expedient the frequency of measurement can reach 10-20 analysis/ hour: this frequency is more than suitable for the normal monitoring of industrial liquid effluents;
e) operating simplicity. The system can work automatically with reduced interventions of the operator: appropriate means of control and realisation can allow the alternate connection of the sampling device with the pipe for the feeding of the sample or with the circuit for the feeding of the solvent;
f) absence of soiling phenomena. The sampling device, the measuring cell of the spectrophotometric detector and the line for the circulation of the solvent are washed with the solvent between two subsequent analyses.

Two additional not negligible advantages with the method object of the present invention consist in the first place in the fact that the method does not require the used of any reactant because it is directly performed on the oil in the organic solvent carrier, and in second place in the possibility to obtain the desired pre-concentration of oils by retention of the same inside the sampling device (with or without packing material) simply by varying the times of circulation of the fluid to be analysed in the sampling device.

A longer contact time between fluid under examination and inside walls of the sampling device translates into an increased quantity of oils retained in the sampling device and therefore in a greater sensitivity of the method.

According to the invention the above described method is carried out by means of an equipment for continuous quantitative measurement of mineral oils in water, characterized in that it comprises a pipe sampling device open at the ends, a first circuit for the feeding and draining of a fluid with mineral oils in water for said sampling device and a second circuit for the feeding and recycling of solvent for the elution of the oils retained inside said sampling device, said second circuit comprising, downstream of said sampling device, a spectrophotometric detector and a device for the regeneration of the solvent, commutation means being provided in order to insert alternately said sampling device in said first or second circuit.

An example of embodiment of the equipment according to the invention is illustrated as a non limiting example in the enclosed drawings, in which:
Figure 1 shows the equipment during the step of feeding of the sample in the sampling device;
Figure 2 shows the equipment during the step of elution and measurement.

More specifically, the equipment shown in the drawings comprises a sampling system 1 consisting of a sampling device 2 and of a commutation device 3. In the rendered embodiment the sampling device 2 is made up of a pipe coil that is open at the ends (realised for example by means of a steel capillary of 0.1÷5 ml in volume and with inside diameter equal to about 0.5 mm, with or without packing material).

Associated with the sampling system 1 is a first circuit 4 for the feeding and draining of the fluid to be analysed, comprising mineral oils in water, that is made circulate by a pump 5 with variable delivery (for example a multi-channel peristaltic pump with six rolls).

In addition the equipment comprises a second circuit 6 for the feeding and recycling of organic solvent, comprising a pump 7 (for example with average or high pressure, with double piston) capable to guarantee a flow of about 1-3 ml/ min, a spectrophotometric detector 8 of the spectrofluorimetric or IR type with in flow analysis cell (with a volume of about 30 µl), a device 9 for the regeneration of the solvent, a solvent tank 10 (with a volume of about 1 l) and a recycle pipe 11. A variable delivery pump 12 for the draining of the water phase (with traces of the solvent) at the end of the operation of analysis is associated with the tank 10. A chart recorder or an integrator 13 and/ or a personal computer 14 are in turn associated with the detector 8. The detector 8 can be of the in flow cell spectrofluorimetric type with adjustable excitation and emission wavelength (for example, 300 nm and 350 nm, respectively) or a detector IR (in which case, using Freon as a solvent, a system that can operate according to the current technical standard is created).

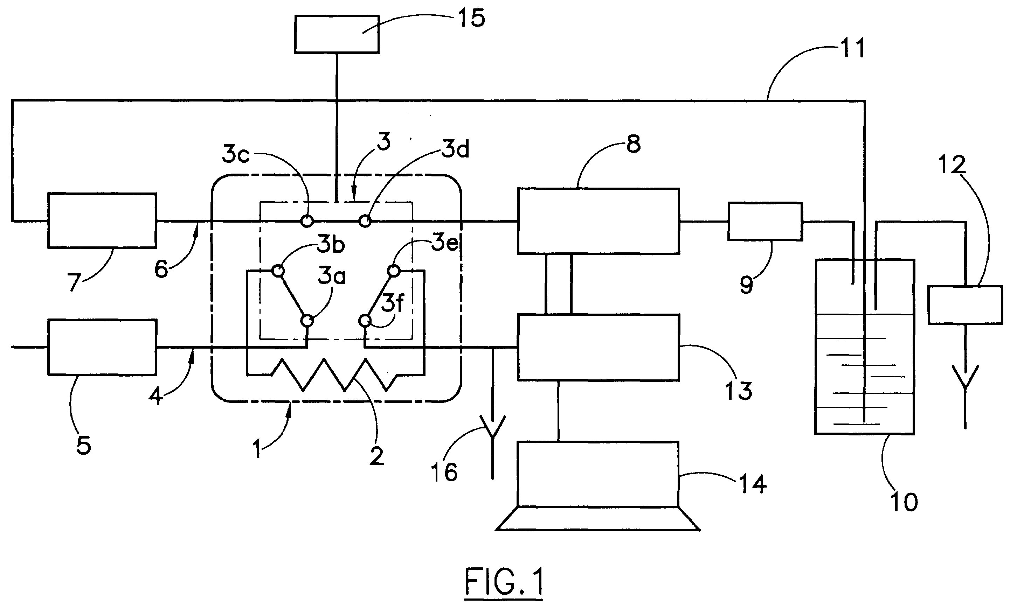

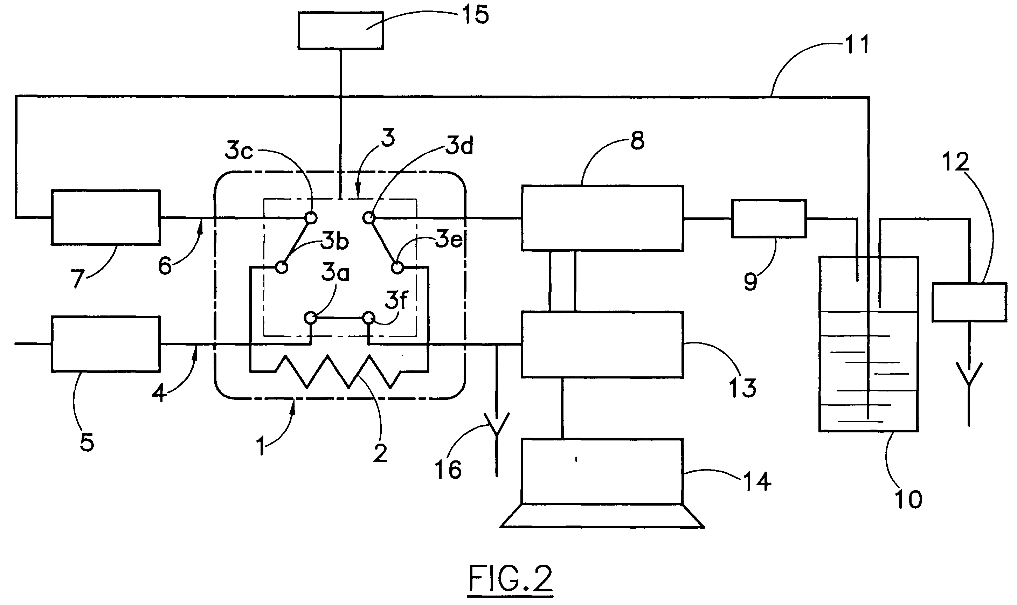

The commutation device 3 can for example consist of a six-way injection valve, respectively designated 3a-3f, that is automatically controlled by control and actuator means 15 that control its alternated commutation in the operating conditions of Figures 1 and 2.

When the valve 3 is in the operating condition of Figure 1, its ways or inlets 3a, 3b are coupled to each other and to the delivery side of the pump 5 of the feeding circuit 4, as also to an end of the sampling pipe coil 2, while the ways or inlets 3e, 3f are coupled to each other and to the other end of the pipe coil 2, as also to a drain 16. The ways or inlets 3c, 3d are finally coupled to each other and to the delivery side of the pump 7 of the circuit 6 for the feeding and recycling of solvent , on one side, and to the spectrophotometric detector 8, on the other side.

With the valve in this operating condition the equipment is in the step of feeding of the fluid to be analysed, that through the circuit 4 and the pump 5 is introduced in the pipe coil 2, where one part of the oils present in the fluid is retained by the walls of the pipe coil while the residual part of the same fluid is conveyed to the drain. In the meantime a suitable solvent (for example an organic n-hexane solvent) is made circulate in the circuit 6 by the pump 7.

Once the step of feeding is over, whose duration depends on the type of fluid to be analysed and on the desired degree of pre-concentration of the oils present, the valve 3 is automatically commuted in the operating condition of Figure 2, where the ways or inlets 3a, 3f are coupled to each other and to the delivery side of the pump 5 , on one side, and to the draining 16, on the other side, the ways or inlets 3b, 3c are coupled to each other and to an end of the pipe coil 2 , on one side, and to the delivery side of the pump 7, on the other side, and the ways or inlets 3d, 3e are coupled to each other and to the other end of the pipe coil 2 , on one side, and to the detector 8, on the other side.

The equipment is now at the step of elution and measurement, in the sense that the solvent, that is made circulate by the pump 7 in the circuit 6, removes the oils retained by the wall of the pipe coil 2 and it transfers them to the detector 8, that carried out the measurement and transmits corresponding signals to the recorder or integrator 13 and from here to the personal computer 14. Downstream of the detector 8 the solvent is regenerated by the regeneration device 9, that is introduced in the solvent tank 10 and from here re-circulated toward the pump suction 7 through the duct 11. In the meantime some possible fluid that is additionally fed by the pump 5 goes directly to the drain or is recycled toward the pump suction 5.

Once the analysis is over, the water phase of the mixed fluid present in the tank 10 (solvent and water) is drained by the pump 12.

## Claims

1. Method for continuous measurement of mineral oils in water, characterized in that it comprises, alternate to each other, a feeding step, in which a fluid to be analysed is pumped into a sampling device (2) in which the oils that are present in the fluid are separated by retention of the same inside said sampling device (2), and an elution and measurement step, in which the oils that are retained in said sampling device (2) undergo elution and are transferred to a spectrophotometric detector (8) by means of circulation of the solvent in a closed circuit (6), comprising said sampling device (2), said spectrophotometric detector (8) and a device for the regeneration (9) of the solvent, in a sequence.

2. Method according to claim 1, characterised in that it employs an organic solvent.

3. Method according to claim 1, characterised in that it provides for the regeneration and the recovery of the solvent mixed with water, the separation of the solvent from the water and the draining of the same water, once the analysis is over.

4. Equipment for continuous quantitative measurement of mineral oils in water, characterized in that it comprises a sampling device (2) with pipe open at the ends, a first circuit (4) for the feeding and draining of a fluid with mineral oils in water for said sampling device (2) and a second circuit (6) for the feeding and recycling of solvent for the elution of the oils retained within said sampling device (2), said second circuit (6) comprising, downstream of said sampling device (2), a spectrophotometric detector (8) and a device for the regeneration (9) of the solvent, commutation means (3) being provided in order to insert alternately said sampling device (2) in said first or second circuit (4, 6).

5. Equipment according to claim 4, characterised in that said sampling device (2) consists of a pipe.

6. Equipment according to claim 5, characterised in that said pipe is realised in the form of a steel capillary.

7. Equipment according to claim 4, characterised in that said commutation means (3) consists in a six-way flow valve, of which the first two ways (3a, 3f) are constantly coupled to a fluid feed in said first circuit (4) and to a drain of the same, two second ways (3b, 3e) are constantly coupled to respective ends of the sampling device (2) and two third ways (3c, 3d) are constantly coupled to a feed of solvent in said second circuit (6) and to said detector (8), said valve being movable from a position of feed (Figure 1), in which said first ways (3a, 3f) are coupled to respective second ways (3b, 3e) and said third ways (3c, 3d) are coupled to each other, at a position of elution and measurement (Figure 2), in which said first ways (3a, 3f) are coupled to each other and said second ways (3b, 3e) are coupled to said third ways (3c, 3d).

8. Equipment according to claim 4, characterised in that said spectrophotometric detector (8) is of the spectrofluorimetric type.

9. Equipment according to claim 4, characterised in that said spectrophotometric detector (8) is an IR detector.

10. Equipment according to claim 4, characterised in that a chart recorder (13) is associated with said spectrophotometric detector (8).

11. Equipment according to claim 4, characterised in that an integrator device (13) is associated with said spectrophotometric detector (8).

12. Equipment according to claim 4, characterised in that a personal computer (14) is associated with said spectrophotometric detector (8).

13. Equipment according to claim 4, characterised in that it comprises a tank (10) for the recovery of the solvent and its separation from water once the analysis is over.

14. Equipment according to claim 13, characterised in that it comprises means (12) for the pumping to drain the water separated from the solvent.
